Europäisches Patentamt

European Patent Office

Office européen des brevets

(19)

(11) Publication number: **0 251 444**

A1

(12) **EUROPEAN PATENT APPLICATION**

(21) Application number: **87303739.4**

(22) Date of filing: **28.04.87**

(51) Int. Cl.³: **B 25 C 5/00**

(30) Priority: **30.04.86 US 857885**

(43) Date of publication of application:
**07.01.88 Bulletin 88/1**

(84) Designated Contracting States:
**DE FR GB IT SE**

(71) Applicant: **MINNESOTA MINING AND MANUFACTURING COMPANY**
3M Center, P.O. Box 33427
St. Paul, Minnesota 55133-3427(US)

(72) Inventor: **Hannula, Donald L**
c/o VIR Engineering 5901 Encing Road
Suite B Goleta California 93117(US)

(72) Inventor: **Vidal, Claude A**
c/o VIR Engineering 5901 Encing Road
Suite B Goleta California 93117(US)

(72) Inventor: **Redmond, Russell J**
c/o VIR Engineering 5901 Encing Road
Suite B Goleta California 93117(US)

(74) Representative: **Baillie, Iain Cameron et al,**
c/o Ladas & Parry Isartorplatz 5
D-8000 München 2(DE)

(54) Anvil assembly.

(57) An anvil (10) against which leg portions (56, 58) of a staple (50) can be pressed to close the staple (50). The leg shaping surfaces include bottom surfaces (16) of sockets (14) including relatively wide initial contact surface portions (20) having widths that smoothly converge toward adjacent final guide portions (24) of a uniform width so that even the legs of staples that contact the initial contact surfaces (20) at positions spaced from the longitudinal center line of the socket (14) will be guided into the final guide portions (24) and bent closed in alignment with each other.

FIG.1 FIG.2

0251444

## Technical Field

The present invention relates to anvils against which generally straight parallel leg portions of a staple projecting at generally a right angle from opposite ends of a generally straight back portion of the staple can be pressed to bend the leg portions into arcuate shapes and form a generally B shaped closed staple.

## Background Art

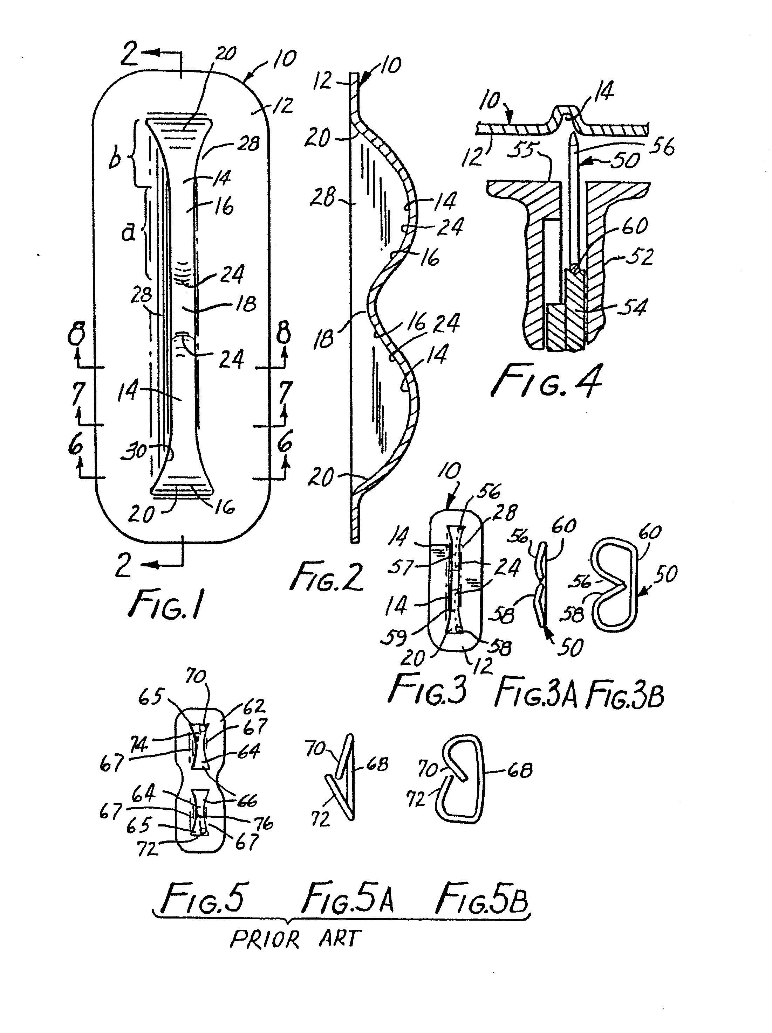

Anvils of many shapes are known against which generally straight parallel leg portions of a staple projecting at generally a right angle from opposite ends of a generally straight back portion of the staple can be pressed to bend the leg portions into arcuate shapes and form a generally B shaped closed staple. Typically such anvils have walls defining a generally planar clamping surface adapted to engage one side of material (e.g., living tissue or paper) through which the leg portions of the staple are driven from the other side of the material, and staple leg shaping surfaces recessed from the clamping surface and defining a pair of elongate sockets aligned end to end, which leg shaping surfaces receive, guide and form the leg portions of the staple to close the staple. A commonly used anvil of this type is illustrated both in U.S. Patent No. 4,281,785 and in Figure 5 of the drawing of this application (to which Figure 5 the following reference numerals refer), which anvil has walls defining such a generally planar clamping surface 62 and staple leg shaping surfaces recessed from the clamping surface 62 and defining a pair of elongate sockets 64 aligned end to end. The leg shaping surfaces for each socket 64 comprise a continuous bottom surface which is concave about axes transverse of the socket 64 and comprises an initial contact portion 65 adjacent its end opposite the other of the sockets 64 and a final guide portion 66 adjacent the other of the sockets 64, and longitudinally extending side surfaces 67 extending between the clamping surface 62 and the bottom surface. The initial contact portions 65 of the bottom surfaces have widths between the side surfaces 67 at their ends opposite the final guide portions 66 that are substantially larger than the diameter of the leg portions 70, 72 of a staple that the anvil is adapted to close, and smoothly converge in width between the side surfaces 67 toward the final guide portions 66 of the bottom surfaces so that leg portions 70, 72 of staples that contact the initial contact portions 65 even at positions spaced from the longitudinal center line of the sockets 64 will be guided into the final guide portions 66. This is an important feature in most stapling devices because the large number of stapling device parts used to position the legs of staples to be driven opposite anvils against which they are to be closed makes the possibility of some misalignment between the staples and anvils quite likely unless the stapling devices are made to unreasonably close and costly tolerances. In an anvil of the type shown in Figure 5, however, the final guide portions 66 of the bottom surfaces diverge in their widths between the side surfaces 67 toward the ends of the bottom surfaces adjacent the other socket 64 so that the leg portions of staples that contact the initial contact portions 65 at positions spaced from the longitudinal center line of the sockets 64, such as at the locations illustrated for the leg portions 70 and 72 in Figure 5, can follow the path of least resistance illustrated by the dotted lines 74 and 76, resulting in an improperly closed staple such as that illustrated in figures 5A and 5B in which the formed leg portions 70 and 72 are not in alignment with each other or with a generally straight central portion 68 of the staple, and the tips of the formed leg portions 70 and 72 are not located as close to the straight central portion 68 of the closed staple as is required to develop the full holding power for the closed staple.

One attempt to correct the problem described above for anvils used to staple living tissues is described in U.S. Patent No. 3,494,533 which describes an anvil 6 which, like the anvil shown in Figure 5 of this application, has walls defining a generally planar clamping surface, and staple leg shaping surfaces 101 (see Figures 28 and 29) recessed from the clamping surface and defining a pair of elongate sockets aligned end to end. The leg shaping surfaces for each socket comprise a continuous bottom surface 101 which is concave about axes transverse of the socket and comprises an initial contact portion 101a adjacent its end opposite the other of the sockets and a final guide portion 101b adjacent the other of the sockets, and longitudinally extending side surfaces extending between the clamping surface and the bottom surface 101. Unlike the anvil described in Figure 5, however, in the anvil described in U.S. Patent No. 4,281,785 both the initial contact portions 101a and the final guide portions 101b of the bottom surfaces 101 have widths between the side surfaces transverse of the sockets that are uniform and only slightly larger than the diameter of the leg portion of a staple the anvil is adapted to close so that the leg portions of a staple should be bent in alignment with each other if the staple leg portions are pressed against the bottom surfaces 101, and inwardly tapered lead in surfaces 103 are provided flanking the initial contact portions 101a that are intended to guide the leg portions of staples that might initially contact the lead in surfaces 103 at positions spaced from the longitudinal center line of the socket to the initial contact portions 101a of the bottom surfaces 101. Such anvil shapes have not as yet seen significant commercial use, however, presumably because the staple legs do not reliably move along the lead in surfaces 103 into the initial contact portions 101a of the bottom surfaces 101, therefore causing such staples to be trapped against the lead in surfaces 103 so that they are deformed and not properly closed.

## Disclosure of the Invention

The present invention provides an anvil against which generally straight parallel leg portions of a staple projecting at generally a right angle from opposite ends of a back portion of the staple can be pressed to bend the leg portions into arcuate shapes and form a generally B shaped closed staple, which anvil will reliably properly guide and form the legs of staples that contact the anvil, including at positions substantially spaced from its center line.

According to the present invention there is provided an anvil which, like the prior art anvil described in U.S. Patent No. 4,281,785 and above with reference to Figure 5 of the drawing, has walls defining a generally planar clamping surface, and staple leg shaping surfaces recessed from the clamping surface and defining a pair of elongate sockets aligned end to end, which leg shaping surfaces for each socket comprise (1) a continuous bottom surface which is concave about axes transverse of the socket and comprises an initial contact portion adjacent its end opposite the other of the sockets and a final guide portion adjacent the other of the sockets, and (2) longitudinally extending side surfaces extending between the clamping surface and the bottom surface. Also like the anvil shown in Figure 5, the initial contact portions have widths between the side surfaces at their ends opposite the final guide portions that are substantially larger than the diameter of the leg portions of a staple that the anvil is adapted to close, and smoothly converge in width between the side surfaces toward the adjacent parts of the final guide portions so that legs of staples that contact the initial contact portions at positions spaced from the longitudinal center line of the socket will be guided into the final guide portions. Unlike the anvil shown in U.S. Patent No. 4,281,785 and in Figure 5, however, the bottom surfaces of the anvil according to the present invention have generally uniform widths between the side surfaces only slightly wider than the diameter of the leg portions of the staple the anvil is adapted to close along all of the final guide portions so that the leg portions of a staple will be bent in alignment with each other for any initial orientation of staple leg portions pressed against the initial contact portions of the bottom surfaces.

Brief Description of Drawing

The present invention will be further described with reference to the accompanying drawing wherein like reference numerals refer to like parts in the several views, and wherein:

Figure 1 is a top plan view of an improved anvil according to the present invention;

Figure 2 is a sectional view taken approximately along line 2-2 of Figure 1;

Figure 3 is a reduced top plan view of the anvil of Figure 1 in which the closing of a staple against the anvil is schematically illustrated;

Figures 3A and 3B are bottom and side views respectively of a staple closed against the anvil of Figures 1,2,and 3 in the manner illustrated in Figure 3;

Figure 4 is a fragmentary sectional view of a stapling assembly including the anvil of Figure 1 and a staple being pressed against the anvil;

Figure 5 is a top plan view of the prior art anvil discussed above in which the closing of a staple against the anvil is schematically illustrated;

Figures 5A and 5B are bottom and side views respectively of a staple closed against the anvil of Figure 5 in the manner illustrated in Figure 5;

Figures 6, 7, and 8 are enlarged fragmentary sectional views taken approximately along lines 6-6, 7-7, and 8-8 of Figure 1 respectively which do not show background details; and

Figure 9 illustrates a structure incorporating a plurality of improved anvils according to the present invention.

Detailed Description

Referring now to the drawing, there is shown an improved anvil according to the present invention generally designated by the reference numeral 10, which anvil 10 may be made of metal or other suitable hard material by known processes such as stamping or coining.

The anvil 10 is of the type against which generally straight parallel leg portions 56 and 58 of a staple 50 projecting at generally a right angle from opposite ends of a generally straight back portion 60 of the staple 50 can be pressed as by a driver 54 in a staple driving mechanism 52 illustrated in Figure 4 to bend the leg portions 56 and 58 into arcuate shapes and form a generally B shaped closed staple as shown in Figures 3A and 3B. The anvil 10 has walls defining a generally planar clamping surface 12 adapted to be positioned opposite and parallel to a cooperating planar clamping surface 55 on the staple driving mechanism 52 between which clamping surfaces 12 and 55 material (e.g., living tissue or paper) to be stapled is held during the stapling process, and staple leg shaping surfaces recessed from the clamping surface 12 and defining a pair of elongate sockets 14 aligned end to end, which sockets 14 are mirror images of each other. The leg shaping surfaces for each socket 14 comprise a continuous bottom surface 16 which is concave about axes transverse of the socket 14 as can be seen in Figure 2 and comprises an initial contact portion 20 adjacent its end opposite the other of the sockets 14, the length of which initial contact portion 20 is identified by a bracket and the reference letter "b" along one of the bottom surfaces 16 shown in Figure 1; and a final guide portion 24 adjacent the other of the sockets 14, the length of which final guide portion 24 is identified by a bracket and the reference letter "a" along one of the bottom surfaces 16 shown in Figure 1. The leg shaping surfaces also include longitudinally extending side surfaces 28 extending between the clamping surface 12 and the bottom surface 16 and converging slightly from the clamping surface 12 toward the bottom surface 16. The initial contact portions 20 have widths between the side surfaces 28 at their ends opposite the final guide portions 24 that are substantially wider than the diameter of the leg portions 56 and 58 of the staple 50 that the anvil 10 is adapted to close, and the widths of the initial contact portions 20 smoothly converge between and with the side surfaces 28 toward the final guide portions 24 with the portions of the side surfaces 28 flanking the initial contact portions 20 being convex so that leg portions 56, 58 of staples that contact the initial contact portions 20 at positions spaced from the longitudinal center line of the sockets 14 such as at the positions illustrated in Figure 3 will be smoothly guided by the initial contact portions 20 and the convex adjacent side surface portions into the final guide portions 24. The bottom surfaces 16 have generally uniform widths between the side surfaces 28 along all of the final guide portions 24, which uniform widths are only slightly wider than the diameter of the leg portions 56 and 58 of the staple 50 the anvil 10 is adapted to close so that the leg portions 56 and 58 of the staple 50 will be guided along the aligned final guide portions 24 and thereby bent in alignment with each other for any initial contact position of the staple leg portions 56 and 58 against the initial contact portions 20 of the bottom surfaces 16 as the staple 50 is pressed against the anvil 10, including the contact positions illustrated in Figure 3 which will result in the staple leg portions 56 and 58 being thus bent into alignment along the paths 57 and 59 illustrated in Figure 3.

The final guide portions 24 of the bottom surfaces 16 defining the two sockets 14 meet at a ridge 18 which could be coplanar with the clamping surface 12, but preferably is recessed from the clamping surface 12 as is illustrated in Figure 2.

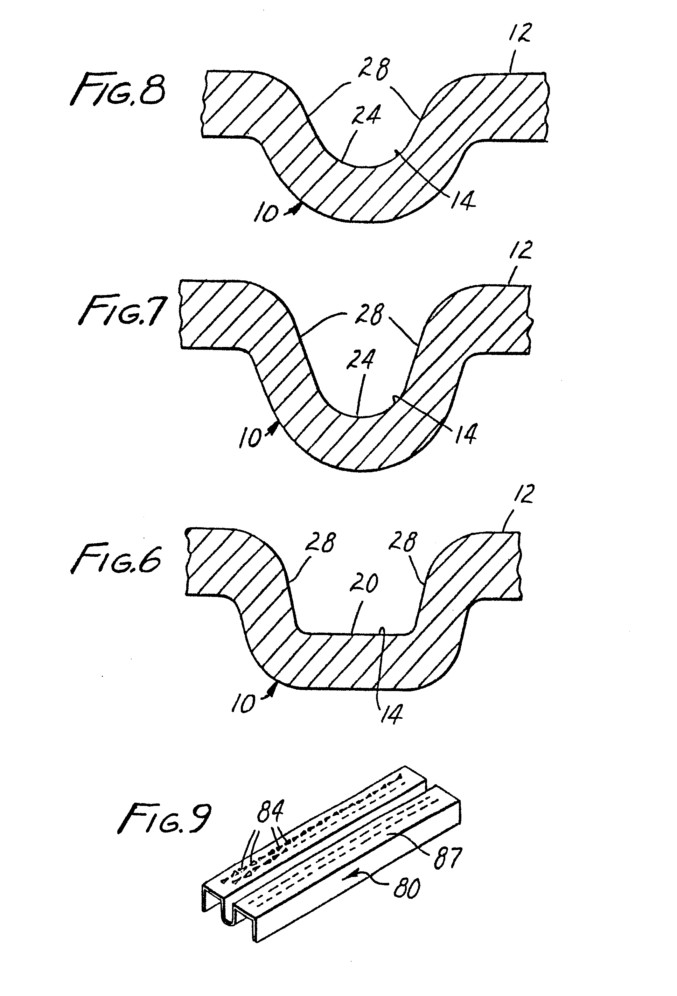

The bottom surfaces 16 defining the two sockets 14 could be planar along their entire length, or, as illustrated and best seen in Figures 6, 7 and 8, the initial contact portions 20 thereof may be planar (Figure 6), and the final guide portions 24 thereof may be generally cylindrically concave (Figures 7 and 8).

Figure 9 illustrates a structure 80 incorporating a plurality of anvils according to the present invention each comprising staple forming surfaces 84 recessed from a common clamping surface 87 and having the same configuration as the staple forming surfaces in the anvil 10. The structure 80 is adapted to close two parallel rows of staples and is of the type used in surgical stapling instruments such as an inverted linear anastomoses surgical stapling instrument. As is apparent, anvils according to the present invention could also be used in surgical stapling instruments that apply staples in many other patterns such as oval or circular.

The present invention has now been described with reference to two embodiments thereof. It will be apparent to those skilled in the art that many changes can be made in the embodiments described without departing from the scope of the present invention. Thus the scope of the present invention should not be limited to the structures descried in this application, but only by structures described by the language of the claims and the equivalents of those structures.

CLAIM:

1. An anvil against which generally straight parallel leg portions of a staple of a predetermined diameter and projecting at generally a right angle from opposite ends of a back portion of the staple can be pressed to bend the leg portions into arcuate shapes and form a generally B shaped closed staple, said anvil having walls defining a generally planar clamping surface, and staple leg shaping surfaces recessed from said clamping surface and defining a pair of elongate sockets aligned end to end, said leg shaping surfaces for each socket comprising a continuous bottom surface which is concave about axes transverse of the socket and comprises an initial contact portion adjacent its end opposite the other of said sockets and a final guide portion adjacent the other of said sockets, and longitudinally extending side surfaces extending between said clamping surface and said bottom surface, said initial contact portions having widths between said side surfaces at their ends opposite said final guide portions that are substantially larger than the the diameter of said leg portions and converge smoothly in width between said side surfaces toward the adjacent parts of said final guide portions so that legs of staples that contact said initial contact portions at positions spaced from the longitudinal center line of said sockets will be guided by said initial contact portions and side surfaces into said final guide portions, charcterized by the feature that said bottom surfaces (16) have generally uniform widths only slightly wider than the diameter of the leg portions (56,58) of said staple (50) between said side surfaces (28) along all of said final guide portions (24) so that the leg portions (56,58) of the staple (50) will be bent in alignment with each other for any initial contact position of the staple leg portions (56,58) against the initial contact portions (20) of said bottom surfaces (16) as the staple (50) is pressed against the anvil (10).

FIG.1

FIG.2

FIG.4

FIG.3  FIG.3A  FIG.3B

FIG.5  FIG.5A  FIG.5B

PRIOR ART

0251444

Fig.8

28

24

12

10

14

Fig.7

28

24

12

10

14

Fig.6

28

20

28

12

10

14

Fig.9

84

87

80

| DOCUMENTS CONSIDERED TO BE RELEVANT | | | EP 87303739.4 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | US - A - 4 281 785 (BROOKS) <br> * Fig. 3 * <br> -- | 1 | B 25 C 5/00 |
| D,A | US - A - 3 494 533 (GREEN) <br> * Fig. 28,29 * <br> -- | 1 | |
| A | US - A - 2 487 565 (LEBER) <br> * Fig. 6 * <br> -- | 1 | |
| A | US - A - 2 073 960 (CROSBY) <br> * Fig. 1,7 * <br> ---- | 1 | |

TECHNICAL FIELDS
SEARCHED (Int. Cl.4)

B 25 C 5/00

B 31 B 1/00

The present search report has been drawn up for all claims

| Place of search <br> VIENNA | Date of completion of the search <br> 07-07-1987 | Examiner <br> KNAUER |
|---|---|---|